# EUROPEAN PATENT APPLICATION

(11) **EP 2 005 874 A1**
(43) Date of publication of application: **24.12.2008**
(21) Application number: 07425387.3
(22) Date of filing: 22.06.2007
(51) Int. Cl.: A61B 1/00, A61M 25/01

(54) **Endoscopic catheter**

(71) Applicant: AMS GROUP S.r.l. Società unipersonale, 35020 San Pietro Viminario, PD (IT)
(72) Inventor: Vendrami, Mauro, c/o AMS Group S.r.l., 35020 S. Pietro Viminaro (PD) (IT)
(74) Representative: Vinci, Marcello

(57) **Abstract**

The invention is a new endoscopic catheter, comprising a handpiece (I) with connection conduits (O), a slender body (F) made of a flexible material and complete with internal channels (Fo1, Fo2) and at least two pairs of wires, the tip (F2) of the catheter being made of a flexible material that enables it to be bent in at least two non-parallel planes.

## Description

The present patent relates to medical instruments and devices and particularly concerns endoscopes with a flexible terminal portion and internal channels for the insertion of means for remote viewing and/or performing surgical procedures and/or administering fluids or gases.

There are known endoscopes, i.e. narrow-gauge catheters for inserting a (usually fiberoptic) viewer for the minimally-invasive visual examination of body cavities.

There are known catheters that are suitably shaped to allow for the passage of surgical instruments to enable minimally-invasive surgical manoeuvres in combination with independent endoscopes.

There are known endoscopic catheters with two or more lumens, at least one of which is used to insert remote viewing means and at least one other of which is used to administer medication, fluids or gases in a specific site inside the body reached by said catheters.

There are known catheters with a flexible distal portion guided by one or more wires for controlling and bending said distal portion, manoeuvred by suitable control means situated on the handpiece of the endoscopic catheter.

Such catheters are also used for periduroscopy or myeloscopy, i.e. for visual diagnostics in areas adjacent to or within the intravertebral spaces in the spinal column.

The periduroscopy and/or myeloscopy technique was developed towards the end of the Eighties and became fundamental to early diagnosis and subsequent treatment procedures, although it initially could not be used to perform any surgical manoeuvres due to the lack of suitable instrumentation. The existing instruments are endoscopic video-guided means developed in the early years of application of periduroscopy and they carry the drawbacks deriving from their use mainly for diagnostic-exploratory purposes: in fact, they have one or two narrow channels or lumens, affording a limited operativity with little potential for development in the area of surgery and micro-morphological diagnostics.

Endoscopic catheters have several drawbacks, however.

The distal end of endoscopic catheters can only be bent in one plane, up and down or side to side, depending on how the catheter is inserted. This restricts its usage as a diagnostic and surgical tool because the areas not covered by the plane in which the tip of the catheter bends are inaccessible both to visual observation and to any surgical manoeuvre.

The catheter cannot be rotated around its central axis in order to bring the catheter tip's bending plane into a different position in which the operator wishes to observe the conditions or take action.

The known endoscopic catheters have couplings or inlets on the handpiece for inserting suitable surgical instruments, bundles of optical fibres for remote viewing, or conduits for delivering fluids or gases. Being located directly on or near the handpiece, these couplings or inlets interfere with the actions of the operators managing and guiding the catheter, and of the assistants taking action to connect the tubes or conduits, or to insert the initial part of the surgical instruments.

A new type of endoscopic catheter has been designed and carried out to overcome all the above-mentioned drawbacks.

One object of the new endoscopic catheter is to enable operators to bend the tip that is inserted in the body in at least two distinct, non-parallel and generically orthogonal planes.

Another object of the new endoscopic catheter is to enable operators to bend the inserted tip independently on several planes.

Another object of the new endoscopic catheter is to make the handpiece easier to handle for the operator, while making it easier and more straightforward for the assistant to insert and withdraw the instruments and conduits in the couplings of the new endoscope.

These and other direct and complementary objects are achieved by the new endoscopic catheter with a flexible tip that can be manoeuvred on two distinct and generically orthogonal planes, controlled by independent cursors on the handpiece and complete with terminal branches and extensions for the operating tubes connected thereto.

The new endoscopic catheter consists of a handpiece with a slender body departing from said handpiece.

Said slender body may consist of a single type of material or of several types of material, at least one of which is a relatively flexible material used to manufacture all or part of the distal tip of said slender body.

The slender body contains at least three operating channels and two supplementary channels for use in lavage and aspiration, and at least two slender control members that are flexible but non-dilatable for the purpose of bending the distal tip of said slender body.

The slender control members are connected either directly or by means of lever mechanisms or other mechanical linkage and constraints to two distinct cursors or sliding levels situated on the handpiece in a readily accessible and manoeuvrable position.

The internal channels of said slender body are connected, inside the handpiece, to suitable conduits for guiding and inserting the instruments. These guiding and inserting conduits emerge from the rear part of the handpiece on different compulsory planes that extend far enough to ensure that the handpiece and the operator's handling of the new endoscopic catheter are sufficiently separate from and unable to interfere with the assistant's insertion of the instruments in said conduits.

The operating channels are designed for the insertion of fibre-optic elements for the remote viewing of the cavity in which the distal end of the slender body of the new endoscopic catheter is inserted, for the insertion of technical operating means, e.g. a Fogarty catheter, for the insertion of biopsy forceps.

The supplementary channels enable lavage and aspiration in the area surrounding the distal end of the slender body of the new endoscopic catheter.

Where necessary, provision can be made for said supplementary channels to enable the passage and positioning of electrodes for measuring and monitoring pressures with the aid of thermistors.

The characteristics of the new endoscopic catheter are better explained in the following description with reference to the drawings, which are attached as nonlimiting examples.

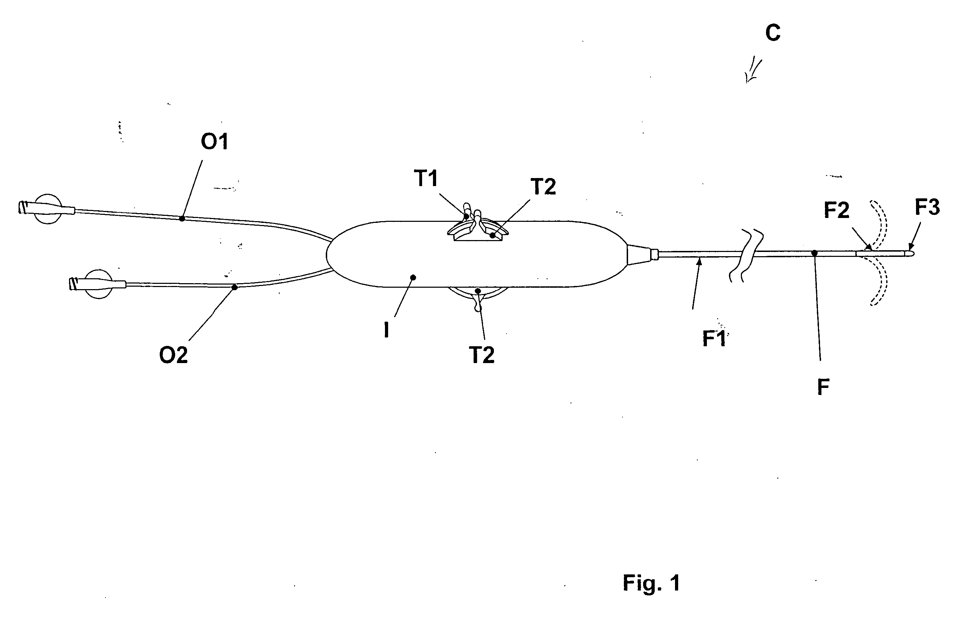

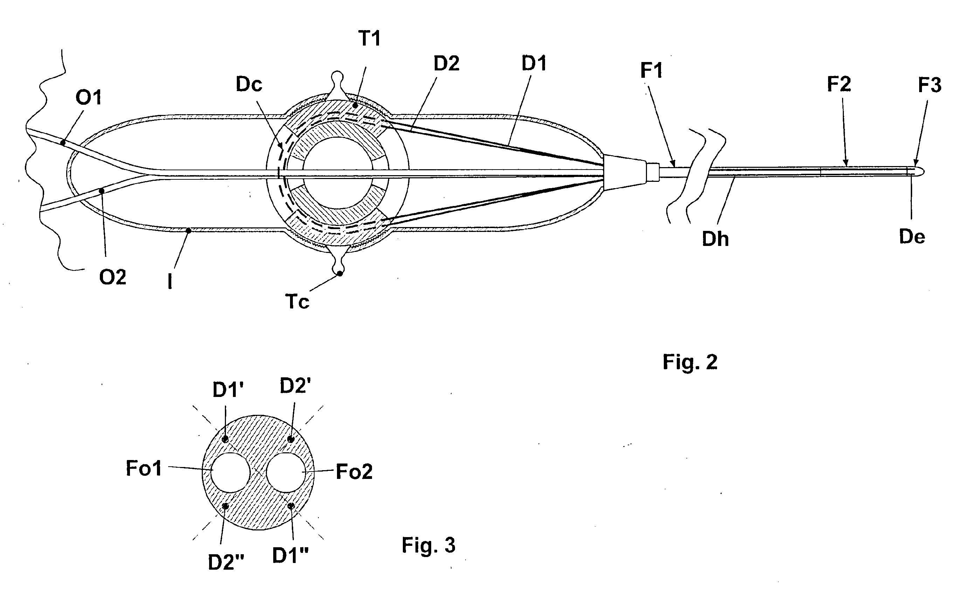

Figure 1 shows a general view of the new endoscopic catheter (C), figure 2 is a cross-section through the inside of the handpiece (I) and the terminal part (F2, F3) of the slender body (F), and figure 3 shows a cross-section of the operating tip (F3) of the catheter (C).

The new endoscopic catheter consists of a handpiece (I) and a slender body (F) departing from said handpiece (I).

The handpiece (I) of the new catheter has a size and shape sufficient to enable it to be held and manoeuvred in one hand with ease, both when the slender body (F) lies on the thumb side and when the slender body lies on the side of the little finger of the operator's hand.

Conduits (O) for connecting the fluid delivery lines and for the insertion of the operating instruments depart from the end of the handpiece (I) opposite the slender body (F).

The slender body (F) has a generically circular cross-section and consists of two materials of different flexibility.

In particular, almost the whole length (F1) of said slender body (F) and the tip (F3) consist of a semi-rigid, or only partly flexible material in order to enable the slender body (F) to be manoeuvred by means of the handpiece (I).

A portion (F2) of the slender body (F) proximal to said tip (F3), hereinafter called the flexible portion (F2) of the slender body (F) for the sake of simplicity, which is a few centimetres long, is made of a flexible material that enables it to be bent easily without its cross-section being substantially modified.

There are channels (Fo1, Fo2) inside the slender body (F) as described above, each of which is attached, inside the handpiece (I) of the catheter (C), to one or more channels (O) for the insertion of instruments and the passage of fluids.

There are two separate controls (T1, T2) on the handpiece, consisting in this example of two wheels lying side by side and projecting from both sides of the thickness of the handpiece (I). The exposed portion of said control wheels (T1, T2) have a relief or appendage (Tc) so that the wheels can be manoeuvred with just one finger of the hand of the operator holding the new catheter (C).

Relatively flexible teeth or other projections are provided on the body of the handpiece (I) and on the surface of said control wheels (T1, T2) so as to maintain each control wheel (T1, T2) in the position assigned by the operator.

There are two control and bending wires (D1, D2), i.e. preferably metal, flexible and non-extendable wires that connect the tip (F3) of the slender body (F) to said control wheels (T1, T2) on the handpiece (I).

In particular, each of said control wires (D1, D2) forms a U shape with its curved portion (Dc) contained in and fixed to a corresponding groove or seat on the part of the control wheel (T1, T2) enclosed in the handpiece (I), while the generically linear parts (Dh) slide along the inside of the handpiece (I) and are contained inside the slender body (F) in the portion not occupied by the channels (Fo1, Fo2), and the ends (De) are permanently attached to the operating tip (F3) of the slender body (F).

The control wires (D1, D2) are arranged inside the slender body (F) so that the two generically linear stretches (Dh) of the same control wire (D1', D1"; D2', D2") lie along a diameter of the cross-section of the slender body (F) and immediately below the outer surface of said slender body (F), and said generically linear stretches (Dh) of the two control wires (D1, D2) lie on two substantially orthogonal diameters of the cross-section of the slender body (F).

The operator taking action on each control wheel (T1, T2) induces a traction on the generically linear stretch (Dh) of the control wire (D1', D2') attached to said control wheel (T1, T2) and a corresponding thrust on the opposite generically linear stretch (Dh) of the same control wire (D1", D2"), thereby making the flexible portion (F2) of the slender body (F) bend towards the side of the linear stretch (Dh) of the control wire (D1, D2) coming under the tensile force.

The traction and thrust on said two generically linear stretches (Dh) of control wire (D1, D2) induce a corresponding traction and thrust on the two opposite sides of the cross-section of the slender body (F), thereby inducing both a modest generic bending of the less flexible stretch (F1) of the slender body (F), and a marked bending of the highly flexible portion (F2) of said slender body (F).

Since the two control wires (D1, D2) lie on two orthogonal planes inside the slender body (F), they induce a bending of said slender body (F), and particularly of its flexible terminal portion (F2), on two planes that are not parallel to one another. The combination and the entity of the tensile force exerted by the control wires (D1, D2) enables the tip (F3) of the slender body (F) of the new catheter to be oriented in any required direction in the area around said tip (F3) of said slender body (F).

The new endoscopic catheter with a flexible tip thus enables the tip to be oriented in all directions around said tip.

The new endoscopic catheter enables the tip to be bent independently on two non-parallel planes.

The new catheter enables the tip to be oriented in all directions around said tip without having to rotate the handpiece.

Thus, with reference to the above description and to the attached drawings, the following claims are advanced.

## Claims

1. Endoscopic catheter, comprising a handpiece (I) with connection conduits (O), a slender body (F) made of a flexible material and complete with internal channels (Fo1, Fo2), having one end (F2) capable of being bent, wherein said conduits (O) in the handpiece (I) are connected to said internal channels (Fo1, Fo2) in the slender body (F), **characterised in that** the portion (F2) of said slender body (F) proximal to the operating tip (F3) is made of a flexible material, and wherein said handpiece (I) comprises devices and/or mechanisms and/or connections suitable for controlling the bending of said flexible portion (F2) of the slender body (F) with the aid of wires lying on at least two non-parallel planes.

2. Endoscopic catheter according to claim 1, **characterised in that** it has two pairs of wires (D1, D2) contained inside the slender body (F), lying along two different diameters, and wherein said wires (D1', D1", D2', D2") forming said pairs (D1, D2) are attached to the distal tip (F3) of said slender body (F) and run through the entire length of the slender body (F), and are fixed or connected to two distinct control members (T1, T2), one for each pair of wires (D1, D2), and wherein each of said control members (T1, T2) is suitable for exerting a tensile force on one or the other of the two respective wires (D1', D1", D2', D2 ").

3. Endoscopic catheter according to claim 2, **characterised in that** each pair of wires (D1, D2) consists of a wire forming a U shape, the linear stretches (Dh) and the ends (De) of which lie inside the slender body (F), while the curved portion (Dc) lies inside the handpiece (I) and is integrally attached to the corresponding control member (T1, T2).

4. Endoscopic catheter according to claim 2, **characterised in that** each control member (T1, T2) consists of a wheel (T1, T2), hinged to and contained partially or entirely inside the handpiece (I), and projecting therefrom on at least one side, and wherein the ends of a pair of wires (D1, D2) are attached to said wheel (T1, T2) along a diameter generically at right angles to the slender body (F).

5. Endoscopic catheter according to claim 3, **characterised in that** each control member (T1, T2) consists of a wheel (T1, T2) contained inside and hinged to the inside of the handpiece (I) and projecting therefrom on at least one side, and wherein the curved portion of the wire (D1, D2) engages in and is fixed to the circumference of, or to a groove within the circumference of said wheel (T1, T2).

6. Endoscopic catheter according to claims 4, 5, **characterised in that** there is a relief or appendage (Tc) on each of said wheels (T1, T2) suitable for manoeuvring even with one finger of the hand of the operator holding the new catheter (C).

7. Endoscopic catheter according to claims 4, 5, **characterised in that** said wheels (T1, T2) are parallel and lie alongside each other.

8. Endoscopic catheter according to claims 4, 5, **characterised in that** each wheel (T1, T2) rotates around an axis at right angles to the bending plane of the tip of the slender body (F) controlled by said wheel.
